## Europäisches Patentamt

## European Patent Office

## Office européen des brevets

(19)

(11) Veröffentlichungsnummer: **0 108 297**
**B1**

(12) # EUROPÄISCHE PATENTSCHRIFT

(45) Veröffentlichungstag der Patentschrift:
**08.01.86**

(51) Int. Cl.⁴: **C 07 C 13/70, C 07 C 1/32**

(21) Anmeldenummer: **83110359.3**

(22) Anmeldetag: **18.10.83**

(54) Verfahren zur Herstellung von (2,2)-Paracyclophan.

(30) Priorität: **30.10.82 DE 3240303**

(43) Veröffentlichungstag der Anmeldung:
**16.05.84 Patentblatt 84/20**

(45) Bekanntmachung des Hinweises auf die Patenterteilung:
**08.01.86 Patentblatt 86/2**

(84) Benannte Vertragsstaaten:
**CH DE FR GB IT LI NL SE**

(56) Entgegenhaltungen:
**ORGANIC SYNTHESIS, collective, Band 5, 1973, Seiten 883-886, John Wiley & Sons, New York, USA**

(73) Patentinhaber: **Merck Patent Gesellschaft mit beschränkter Haftung, Frankfurter Strasse 250, D-6100 Darmstadt (DE)**

(72) Erfinder: **Härtner, Hartmut, Dr., Mathildenweg 9, D-6109 Mühltal (DE)**

## Beschreibung

Die Erfindung betrifft ein neues verbessertes Verfahren zur Herstellung von [2,2]-Paracyclophan (I).

I ist eine bekannte Substanz, die als Zwischenprodukt u.a. zur Herstellung von Poly-p-xylylen verwendet werden kann.

Zur Herstellung von I sind mehrere Verfahren beschrieben, die jedoch für eine technische Produktion erhebliche Nachteile aufweisen.

So werden bei der Hofmann-Eliminierung aus p-Methylbenzyltrimethylammoniumhydroxid (II) in Gegenwart einer Base (vgl. Organic Syntheses, Coll. Vol. 5, John Wiley & Sons, Inc., New York/London/Sydney/Toronto, 1973, Seiten 883-886; Dissertation R. Näder, Univ. Göttingen, 1978, Seite 89) sowie bei der Pyrolyse von p-Xylol mit Wasserdampf bei etwa 900° C nur geringe Ausbeuten (maximal sind 17-19% angegeben) erhalten; daneben entstehen mehr oder weniger unerwünschte polymere Produkte (bis zu 80%).

Bei der 1,6-Eliminierung von (p-Trimethylsilyl-methylbenzyl)-trimethylammoniumjodid mit Tetrabutylammoniumfluorid (vgl. J. Org. Chem., Band 46, 1981, Seiten 1043-1044) liegt die Ausbeute zwar höher (56%); jedoch sind die Ausgangsstoffe schwer zugänglich und teuer, so dass dieses Verfahren für eine technische Herstellung nicht in Frage kommt.

Der Erfindung lag die Aufgabe zugrunde, ein verbessertes Verfahren zur Herstellung von I aufzufinden, das die Nachteile der bekannten Verfahren nicht oder nur in geringerem Masse aufweist und insbesondere zu höheren Ausbeuten führt.

Es wurde nun gefunden, dass die Ausbeute bei der genannten Hofmann-Eliminierung auf 70% gesteigert werden kann, wenn man als Base NaOH oder KOH verwendet und in Gegenwart von Dimethylsulfoxid (DMSO) arbeitet.

Dieses Ergebnis war überraschend, da ein Zusatz anderer vergleichbarer aprotischer Lösungsmittel wie Dimethylformamid, N-Methylpyrrolidon oder Sulfolan wirkungslos ist.

Gegenstand der Erfindung ist ein Verfahren zur Herstellung von [2,2]-Paracyclophan durch Hofmann-Eliminierung aus p-Methylbenzyltrimethylammoniumhydroxid in Gegenwart einer Base, dadurch gekennzeichnet, dass man als Base Natrium- oder Kaliumhydroxid in Gegenwart von Dimethylsulfoxid verwendet.

Besonders vorteilhaft ist es, konzentrierte wässerige Natronlauge oder wässerige Kalilauge sowie ein zusätzliches inertes Lösungsmittel zu verwenden. Als Lösungsmittel eignen sich insbesondere Kohlenwasserstoffe, bevorzugt Toluol oder Xylol, aber auch Benzol oder Tetralin. Die Reaktionstemperaturen liegen zweckmässig zwischen 50 und 130, vorzugsweise zwischen 75 und 95° C.

Der Ausgangsstoff II wird zweckmässig in situ durch Einwirkung der Base auf ein entsprechendes quartäres Salz, vorzugsweise p-Methylbenzyltrimethylammoniumchlorid (III) oder -bromid, gebildet.

Eine besonders bevorzugte Arbeitsweise besteht darin, dass man etwa 45-50%ige Natronoder Kalilauge mit Toluol und DMSO versetzt, dann unter Rühren bei etwa 75 bis 95° C eine konzentrierte wässerige Lösung des Salzes (z.B. III) hinzutropft und etwa 10-100 Stunden weiter unter Rühren erhitzt. Unter diesen Bedingungen ist die Aufarbeitung besonders einfach.

### Beispiel 1

Zu einer Lösung von 210 g NaOH in 240 ml wasser gibt man 3 l Toluol und 600 ml DMSO, sodann tropfenweise unter Rühren bei 90° innerhalb 1 Stunde eine Lösung von 150 g III in 90 ml Wasser. Intermediär entsteht II, das nicht isoliert wird. Man rührt noch 40 Stunden bei 90°, trennt die Phasen, wäscht die organische Phase mit Wasser, filtriert, konzentriert die Lösung und erhält 55 g [2,2]-Paracyclophan, F. 275°.

### Beispiel 2

Man arbeitet analog Beispiel 1, verwendet jedoch 294 g KOH an Stelle des NaOH, und erhält ein vergleichbares Ergebnis.

## Patentanspruch

Verfahren zur Herstellung von [2,2]-Paracyclophan durch Hofmann-Eliminierung aus p-Methylbenzyltrimethylammoniumhydroxid in Gegenwart einer Base, dadurch gekennzeichnet, dass man als Base Natrium- oder Kaliumhydroxid in Gegenwart von Dimethylsulfoxid verwendet.

## Claim

A process for the preparation of [2,2]-paracyclophane, by Hofmann elimination, from p-methylbenzyltrimethylammonium hydroxide in the presence of a base, characterized in that sodium hydroxide or potassium hydroxide is used as the base in the presence of dimethyl sulfoxide.

## Revendication

Procédé de préparation du [2,2]-paracyclophane par élimination de Hofmann à partir de l'hydroxyde de p-méthylbenzyltriméthylammonium en présence d'une base, caractérisé en ce que l'on utilise en tant que base l'hydroxyde de sodium ou l'hydroxyde de potassium en présence de diméthylsulfoxyde.